(19) Europäisches Patentamt
European Patent Office
Office européen des brevets

(11) Veröffentlichungsnummer: **0 068 302**
**A1**

# (12) EUROPÄISCHE PATENTANMELDUNG

(21) Anmeldenummer: **82105264.4**

(22) Anmeldetag: **16.06.82**

(51) Int. Cl.³: **C 07 D 487/04,** A 61 K 31/505
// (C07D487/04, 239/00, 235/00)

(30) Priorität: **24.06.81 DE 3124718**

(43) Veröffentlichungstag der Anmeldung: **05.01.83**
**Patentblatt 83/1**

(84) Benannte Vertragsstaaten: **AT BE CH DE FR GB IT LI LU NL SE**

(71) Anmelder: **BOEHRINGER INGELHEIM KG, ZA Patente, D-6507 Ingelheim am Rhein (DE)**

(72) Erfinder: **Stähle, Helmut, Dr., Rotweinstrasse 23, D-6507 Ingelheim/Rhein (DE)**
Erfinder: **Köppe, Herbert, Dr., Neuweg 72, D-6507 Ingelheim/Rhein (DE)**
Erfinder: **Kummer, Werner, Dr., Georg-Scheuing-Strasse 15, D-6507 Ingelheim/Rhein (DE)**
Erfinder: **Stockhaus, Klaus, Dr., Pfarrer-Heberer-Strasse 35, D-6530 Bingen (DE)**
Erfinder: **Hoefke, Wolfgang, Dr., Rosselstrasse 21, D-6200 Wiesbaden (DE)**
Erfinder: **Galda, Wolfram, Dr., Paul-Clemen-Strasse 14, D-6507 Ingelheim/Rhein (DE)**

(54) **Neue Imidazo(1,2-a)pyrimidine, deren Säureadditionssalze, diese enthaltende Arzneimittel und Verfahren zu deren Herstellung.**

(57) Die Erfindung betrifft neue Imidazo[1,2-a]pyrimidine der allgemeinen Formel

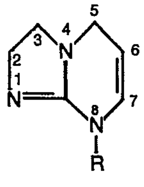

I

worin R einen ein- oder mehrfach durch ein Wasserstoff-, Fluor-, Chlor- oder Bromatom, eine Methyl- oder Trifluormethylgruppe substituierten Phenylrest bedeutet sowie deren Säureadditionssalze.

Die neuen Verbindungen können durch Cyclisierung von Verbindungen der Formel II

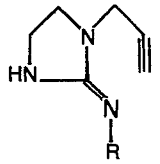

II

hergestellt werden und sind als Analgetika, Antihypertonika und Herz- und Coronartherapeutika verwendbar.

EP 0 068 302 A1

ACTORUM AG

COMPLETE DOCUMENT

Die Erfindung betrifft neue substituierte Imidazo[1,2-a]pyrimidine der allgemeinen Formel

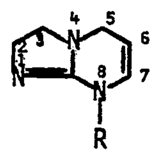

I

sowie deren physiologisch verträgliche Säureadditionssalze mit wertvollen therapeutischen Eigenschaften. In der Formel I bedeutet R einen ein- bis dreifach substituierten Phenylrest. Die Substituenten, welche gleich oder verschieden sein können, bedeuten ein Wasserstoff-, Fluor-, Chlor- oder Bromatom oder eine Methyl- oder Trifluormethylgruppe.

Die Herstellung der Imidazo[1,2-a]pyrimidine der allgemeinen Formel I erfolgt durch
a) thermische Cyclisierung von Verbindungen der allgemeinen Formel II

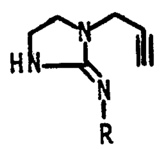

II

worin der Rest R wie oben angegeben definiert ist, bei Temperaturen zwischen 60 und 180°C; oder
b) Isomerisierung von Imidazo[1,2-a]pyrimidinen der allgemeinen Formel III

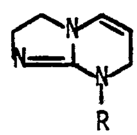

III

worin der Rest R wie oben angegeben definiert ist, in Gegenwart einer starken Base, bei Temperaturen von 40 bis 60°C.

Die thermische Cyclisierung nach Verfahren a erfolgt zweckmäßigerweise durch Erhitzen der Verbindungen der allgemeinen Formel II in Gegenwart eines polaren oder unpolaren organischen Lösungsmittels auf Temperaturen von etwa 60 bis 180°C.
Die speziellen Temperaturen hängen von der Reaktivität der jeweiligen Verbindung der Formel II ab.

Bei Verfahren a entstehen neben den erfindungsgemäßen Verbindungen der allgemeinen Formel I zusätzlich auch deren isomere Verbindungen der allgemeinen Formel IV.

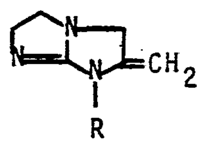

IV

(R ist wie oben definiert)

die abgetrennt werden müssen.

Da sich die Isomeren der allgemeinen Formeln I und IV in ihren pk-Werten deutlich voneinander unterscheiden ($pk_I > pk_{IV}$), lassen sie sich aufgrund dieses Unterschiedes gut voneinander trennen. Durch fraktioniertes Extrahieren der wäßrigen Lösung eines Gemisches von Verbindungen der Formeln I und IV bei aufsteigenden pH-Werten können die schwächer basischen Verbindungen der allgemeinen Formel IV zuerst extrahiert werden, während die stärker basischen, erfindungsgemäßen Verbindungen der allgemeinen Formel I noch in der wäßrigen Lösung verbleiben. Die Substanzen mit

dem größten pk-Wert, also die erfindungsgemäßen Verbindungen der allgemeinen Formel I, zeigen bei der Dünnschichtchromatografie (Kieselgel) in den Systemen

A = Toluol/Dioxan/Äthanol/konz. Ammoniak (50 : 40 : 5 : 5)
B = Essigester/Isopropanol/konz. Ammoniak (70 : 50 : 20)
C = sek.-Butanol/Ameisensäure (85 %)/$H_2O$ (75 : 15 : 10)
einen tieferen $R_F$-Wert als die isomeren Verbindungen der allgemeinen Formel IV. Die erfindungsgemäßen Verbindungen lassen sich nach weiterem Alkalisieren der wäßrigen Lösung z.B. mit Natronlauge auf einen höheren pH-Wert ebenfalls in reiner Form extrahieren und somit isolieren,wobei die Reinheit der Extrakte durch Dünnschichtchromatografie geprüft werden kann.

Bei der Isomerisierung der Verbindungen der allgemeinen Formel III (Verfahren b) arbeitet man zweckmäßigerweise in polaren aprotischen Lösungsmitteln in Gegenwart einer starken Base, z.B. Kalium-tertiär-butylat sowie bei erhöhter Temperatur, beispielsweise 40 bis 60°C.

Die Strukturen der neuen Imidazo[1,2-a]pyrimidine der allgemeinen Formel I sind durch $^1H$- bzw. $^{13}C$-Kernresonanz und massenspaktroskopische Untersuchungen gesichert.

Ausgangsverbindungen der allgemeinen Formel II sind bekannt und z.B. in der DE-OS 25 23 103 beschrieben.

Ausgangsverbindungen der allgemeinen Formel III können durch thermische Cyclisierung von 2-[N-(Subst.-phenyl)-N-propargyl-amino]-2-imidazolinen bei Temperaturen von 60 bis 180°C hergestellt werden.

Die erfindungsgemäßen Imidazo[1,2-a]pyrimidine der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken analgetisch, blutdrucksenkend und herzfrequenzsenkend. Die analgetische Wirkung wurde nach dem Writhing-Test an der Maus geprüft. Darüber hinaus wurde bei der blutigen Messung des Blutdrucks am Kaninchen eine blutdrucksenkende Wirkung festgestellt. Die Beeinflussung der Herzfrequenz wurde an Spinalratten sowie intakten narkotisierten Ratten untersucht. Aufgrund dieser Wirkungen kommen die Verbindungen der allgemeinen Formel I als Arzneimittel zur Behandlung von Schmerzzuständen, der Hypertonie und von Coronarerkrankungen in Frage. Die Wirkstoffe können enteral oder parenteral verabreicht werden. Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele sollen die Erfindung und die Herstellung der neuen Verbindungen erläutern, ohne sie zu beschränken.

Herstellungsbeispiele

Beispiel 1

8-(2,6-Dichlorphenyl)-2,3,5,8-tetrahydro-imidazo[1,2-a]pyrimidin

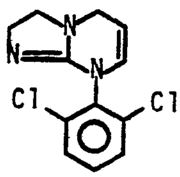

5,4 g 1-Propargyl-2-(2,6-dichlorphenylimino)-imidazolidin werden in 40 ccm Äthanol 10 Stunden lang unter Rühren am Rückfluß erhitzt. Sodann wird die Reaktionsmischung im Vakuum zur Trockne eingedampft. Der Rückstand wird in 1 N Salzsäure gelöst und die erhaltene Lösung bei aufsteigenden pH-Werten (Alkalisieren mit 2 N NaOH) fraktioniert mit Äther extrahiert. Das Ausgangsimidazolidin sowie eine isomere Verbindung der Formel IV können durch Extraktion bei niederen pH-Werten abgetrennt werden (Dünnschichtchromatogramm-Kontrolle). Sobald das gewünschte Imidazo[1,2-a]-pyrimidin in der wäßrigen Phase rein vorliegt, alkalisiert man mit 2 N Natronlauge nach und extrahiert die neue Verbindung mit Äther (Dünnschichtchromatogramm-Kontrolle der Ätherfraktionen). Nach Abziehen des Äthers im Vakuum erhält man eine Ausbeute von 1,1 g entsprechend 20,5 % der Theorie.
Schmelzpunkt: 156 bis 166°C.
Das Hydrobromid schmilzt bei 203 bis 204°C.

Beispiel 2

8-(2-Brom-6-fluor-phenyl)-2,3,5,8-tetrahydro-imidazo-[1,2-a]pyrimidin

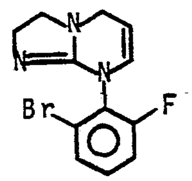

8,9 g 1-Propargyl-2-(2-brom-6-fluorphenylimino)-imidazolidin werden in 60 ml absolutem Äthanol etwa 11 Stunden lang am Rückfluß erhitzt. Anschließend zieht man das Lösungsmittel im Vakuum ab und löst den verbleibenden Rückstand, bestehend aus Ausgangsimidazolidin, 1-(2-Brom-6-fluor-phenyl)-2,3,5,6-tetrahydro-2-methylen-1H-imidazo-[1,2-a]imidazol sowie dem gewünschten Imidazo[1,2-a]-pyrimidin, in verdünnter 1N Salzsäure.

Bei aufsteigenden pH-Werten (Alkalisieren mit 2 N NaOH) entfernt man sodann durch fraktionierte Extraktion mit Essigester die beiden zuerst erwähnten Verbindungen. Sobald die wäßrige Lösung nur noch das neue Imidazo[1,2-a]pyrimidin enthält (Dünnschichtchromatogramm-Kontrolle) wird erneut alkalisiert und die neue Verbindung mit Essigester extrahiert.
Ausbeute:(nach dem Einengen im Vakuum) : 0,9 g (10,1 % der Theorie)
Schmelzpunkt: 116 bis 120°C.

Beispiel 3

8-(2-Chlor-6-methyl-phenyl)-2,3,5,8-tetrahydro-imidazo-[1,2-a]pyrimidin

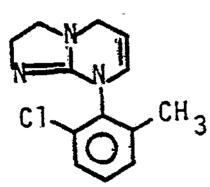

620 mg 8-(2-Chlor-6-methyl-phenyl)-2,3,7,8-tetrahydro-imidazo[1,2-a]pyrimidin werden in 5 ccm Dimethylsulfoxid gelöst und dieser Lösung 280 mg Kalium-tert.-butylat unter Rühren zugegeben. Die Mischung wird sodann 48 Stunden bei 50 bis 60°C erwärmt. Nach dieser Zeit ist ein Teil des eingesetzten 2,3,7,8-Tetrahydro-imidazo[1,2-a]pyrimidins zum 2,3,5,8-Tetrahydro-imidazo[1,2-a]pyrimidin isomerisiert. Eine Trennung kann nach Eindampfen des Lösungsmittels und nach Lösen des vorliegenden Isomeren-Gemisches in verdünnter 1N HCl Salzsäure, wie bei den Beispielen Nr. 1 und 2 beschrieben, durch fraktionierte Extraktion bei aufsteigenden pH-Werten vorgenommen werden (Dünnschichtchromatogramm-Kontrolle).
Schmelzpunkt: 107 bis 113°C.

Weitere Beispiele von Verbindungen der allgemeinen Formel I finden sich in der folgenden Tabelle:

| Beispiel Nr. | R | Ausbeute (% der Theorie) | Schmp. (°C) |
|---|---|---|---|
| 4 | 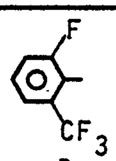 | 12,0 | 115-117 |
| 5 | 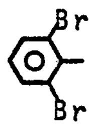 | 22,4 | 144-146 |
| 6 | 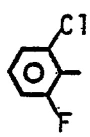 | 18,9 | 146-151 |
| 7 | 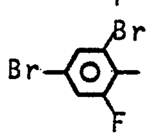 | 17,8 | 104-108 |

| Beispiel Nr. | R | Ausbeute (% der Theorie) | Schmp. (°C) |
|---|---|---|---|
| 8 | 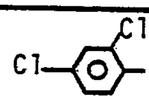 | 19,6 | 91-94 |
| 9 | 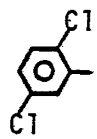 | 16,0 | 113-117 |
| 10 | 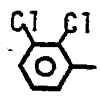 | 26,0 | 134-138 |
| 11 | 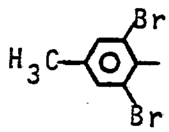 | 21,9 | 120-123 |

## Formulierungsbeispiele

### Beispiel A: Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:
Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Drageekernen von 250 mg Gewicht verpreßt, die dann in üblicher Weise mit Hilfe von Zucker, Talkum und Gummi arabicum dragiert werden.

### Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung:
Wirkstoff und Natriumchlorid werden in Wasser gelöst und unter Stickstoff in Glasampullen abgefüllt.

### Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

## PATENTANSPRÜCHE:

1. Neue Imidazo[1,2-a]pyrimidine der allgemeinen Formel

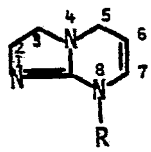

I

worin R einen ein- bis dreifach durch ein Wasserstoff-, Fluor-, Chlor- oder Bromatom, eine Methyl- oder Trifluormethylgruppe substituierten Phenylrest, wobei die Substituenten gleich oder verschieden sein können, bedeutet sowie deren Säureadditionssalze.

2. Verfahren zur Herstellung von Verbindungen nach Anspruch 1, dadurch gekennzeichnet, daß
a) eine Verbindung der allgemeinen Formel

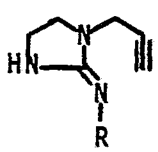

II

worin R wie oben angegeben definiert ist, bei Temperaturen von 60 bis 180°C cyclisiert; oder
b) ein Imidazo[1,2-a]pyrimidin der allgemeinen Formel

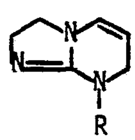

III

worin R wie oben angegeben definiert ist, in Gegenwart einer starken Base bei Temperaturen von 40 bis 60°C isomerisiert und gegebenenfalls die erhaltene Base in ein Säureadditionssalz überführt.

3. Verfahren nach Anspruch 2, dadurch gekennzeichnet, daß man die Umsetzung in Gegnewart eines polaren oder unpolaren organischen Lösungsmittels durchführt.

4. Pharmazeutische Zubereitungen, dadurch gekennzeichnet, daß sie als Wirkstoff eine oder mehrere Verbindungen nach Anspruch 1 enthalten.

5. Verfahren zur Herstellung von Arzneimitteln nach Anspruch 4, dadurch gekennzeichnet, daß man eine oder mehrere Verbindungen nach Anspruch 1 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

6. Verwendung von Verbindungen nach Anspruch 1 bei der Bekämpfung von Schmerzzuständen, Hypertonien und von Herz- und Coronarerkrankungen.

Europäisches Patentamt

# EUROPÄISCHER RECHERCHENBERICHT

0068302

Nummer der Anmeldung

EP 82 10 5264

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 118 261 (BOEHRINGER)<br>* Ansprüche 1,6 *<br>----- | 1,4 | C 07 D 487/04<br>A 61 K 31/505//<br>(C 07 D 487/04<br>C 07 D 239/00<br>C 07 D 235/00) |
| | | | **RECHERCHIERTE SACHGEBIETE (Int. Cl. 3)** |
| | | | C 07 D 487/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 01-10-1982 | ALFARO I. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82